# EUROPÄISCHE PATENTSCHRIFT

(11) **EP 3 207 956 B1**
(45) Veröffentlichungstag und Bekanntmachung des Hinweises auf die Patenterteilung: **05.09.2018**
(21) Anmeldenummer: 17151704.8
(22) Anmeldetag: 17.01.2017
(51) Int. Cl.: A61N 1/02, H01R 43/24, H01B 7/04, H01R 43/00, B29C 65/78, B29C 65/00, H01B 13/00, A61N 1/05, A61M 25/00, A61B 5/00

(54) **VERFAHREN ZUR HERSTELLUNG EINER ELEKTRODENLEITUNG ODER EINES KATHETERS**
METHOD FOR PRODUCING AN ELECTRODE LEAD OR A CATHETER
PROCÉDÉ DE FABRICATION D'UNE CONDUITE D'ÉLECTRODES OU D'UN CATHÉTER

(30) Priorität: 29.01.2016 DE 102016101619
(43) Veröffentlichungstag der Anmeldung: 23.08.2017
(73) Patentinhaber: BIOTRONIK SE & Co. KG, 12359 Berlin (DE)
(72) Erfinder: Bihler, Eckardt, 8406 Winterthur (CH); Hauer, Marc, 8610 Uster (CH); Toelke, René, 8055 Zuerich (CH)
(74) Vertreter: Keck, Hans-Georg

(56) Entgegenhaltungen:
- WO-A1-2015/172729
- DE-A1- 4 301 692
- DE-T2- 60 214 789
- US-A- 4 589 584
- US-A1- 2010 037 457

## Beschreibung

Die Erfindung betrifft ein Verfahren zur Herstellung einer medizinischen Elektrodenleitung oder eines Katheters.

### Technologischer Hintergrund

Im Bereich der Elektrotechnik ist eine Vielzahl von Technologien entwickelt worden, mit der ein elektrischer Kontakt dauerhaft hergestellt werden kann. Unter Kontaktierung wird dabei eine sichere elektrische Verbindung zwischen zwei Leitern verstanden. Die Aufgabe einer elektrischen Verbindung ist somit die Leitung eines elektrischen Stromes über diese Kontaktstelle. Dabei ergibt sich naturgemäß ein Übergangswiderstand, der normalerweise so klein wie möglich sein soll.
Die elektrische Verbindungstechnik ist ein elementarer Bestandteil zur Herstellung von elektrischen Baugruppen und letztendlich elektrischen Geräten und sorgt für die Verbindung der Bauelemente untereinander sowie für die Anschlusstechnik zur Außenwelt. Ebenso findet sich innerhalb von Bauelementen teilweise eine Vielzahl von elektrischen Kontaktstellen.
Unlösbare elektrische Verbindungen beziehungsweise Verbindungstechnologien umfassen beispielsweise:
- Schweißungen (Schweißen)
- Bondverbindungen, Bonden
- Klebverbindungen, Kleben (mit Leitkleber)
- Pressverbindung, Quetschverbinder, Stoßverbinder
   (zum Beispiel mittels Kabelschuhen oder Presshülsen)
- Nietungen

Eine weitere Unterteilung kann in Lötverbindungen und lötfreie Verbindungen erfolgen. Für medizinische Produkte, die für die intrakorporale Anwendung vorgesehen sind, sollten vorzugsweise lötfreie Verbindungen erstellt werden.

Je nach Auslegung in der Designphase ist die Lösbarkeit beziehungsweise die bedingte Lösbarkeit bei größeren und teureren Geräten vorgesehen. Bei Produkten für den einmaligen Gebrauch richtet sich das gewählte Verbindungsverfahren eher nach dem preiswertesten Herstellverfahren für den Produzenten und berücksichtigt die Reparaturfähigkeit kaum noch. Zu letzterer Produktpalette gehören Katheter und Elektroden für die medizinische Anwendung, in denen insbesondere flexible Materialien zum Einsatz kommen. So beinhalten diese Produkte in der Regel kabelförmige Bestandteile, in denen eine oder mehrere Leiter in einem flexiblen Isolator eingebettet sind. Die Herstellung solcher flexibler Leiterabschnitte für Katheter und Elektroden unterliegt derzeit fertigungsbedingt einer Längenbeschränkung. Kabelabschnittslängen ab etwa 60 cm und mehr müssen aus diesem Grunde durch Zusammenstellung mehrerer Teilabschnitte erstellt werden. Die elektrische Verbindung der einzelnen Teilabschnitte muss dauerhaft und zuverlässig sein und sollte nur biokompatible Materialien verwenden. Es besteht daher ein anhaltender Bedarf nach einer kostengünstigen und für die medizinische Anwendung geeigneten Verbindungstechnologie.

Die US-Patentanmeldung US20100037457A1 beschreibt ein Verfahren um zwei Halbzeuge, bestehend aus Helixleiter mit Isolation, zu einer Leitung zu verbinden. Das US-Patent US4589584A beschreibt ebenfalls ein Verfahren zur Verbindung zweier Flachbandkabel. Die Patentanmeldung DE000004301692A1 zeigt ein Verfahren um eine auf einer Platte angeordnete Leiterbahn mit einer anderen, auf einer weiteren Platte angeordneten Leiterbahn unter Anwendung von Ultraschallwellen und Druck zu verbinden. Die PCT-Anmeldung WO2015172729A1 beschäftigt sich mit einem Verfahren zur Herstellung eines Verbindungselementes für Leiterplatten.

Dokument DE60214789T2 beschreibt ein Flachkabel das so aufgebaut ist, dass mehrere flache, rechteckige Leiter in dessen Breitenrichtung angeordnet sind und die Oberflächen der Leiter durch ein Isoliermaterial bedeckt sind. Am Endabschnitt des Flachkabels ist das an den Leiteroberflächen angeordnete Isoliermaterial an einer Seite entfernt, um dadurch an einer Seite Außenflächen der Anschlussendabschnitte der jeweiligen Leiter nach außen hin freizulegen.
Ein oder mehrere Nachteile des Standes der Technik werden mit Hilfe des erfindungsgemäßen Verfahrens zur Herstellung einer Elektrodenleitung oder eines gemäss Anspruch 1 gelöst oder zumindest gemindert.
Das erfindungsgemäße Verfahren zur Herstellung einer Elektrodenleitung oder eines Katheters umfasst die folgenden Schritte:
a) Bereitstellen eines ersten und zweiten Halbzeugs, wobei jedes Halbzeug ein Kabel mit zumindest einem elektrischen Leiter umfasst, der in einem Mantel aus einem elektrisch isolierenden, thermoplastischen Material eingebettet ist und dadurch gekennzeichnet ist, dass das Kabel an zumindest einem Kabelende eine Verbindungsstelle ausbildet, über die der Leiter dauerhaft und unlösbar an weitere Komponenten anschließbar ist, wobei
   - ein Teil der Oberfläche des Leiters im Bereich der Verbindungsstelle freiliegt und eine Kontaktfläche ausbildet und
   - der Mantel im Bereich der Verbindungsstelle eine in Längsrichtung des Kabels durchgehende Ausnehmung aufweist,
   - und wobei eine Kontur der Ausnehmungen der Verbindungsstelle des ersten Halbzeugs im Wesentlichen einer Kontur des Mantels im Bereich der Verbindungsstelle des zweiten Halbzeugs entspricht;
b) Positionieren der beiden Halbzeuge in einem Werkzeug derart, dass die Verbindungsstellen passend aufeinanderliegen, so dass sich die Kontaktflächen der beiden Leiter berühren und die Konturen der Verbindungstellen der beiden Halbzeuge ineinandergreifen;
c) Thermisches Verschmelzen des thermoplastischen Materials im Bereich der Verbindungsstellen.

Der Erfindung liegt die Erkenntnis zugrunde, dass sich eine zuverlässige elektrische Verbindung der Einzelkomponenten einer flexiblen medizinischen Elektrode oder eines flexiblen Katheters mit Hilfe spezifisch ausgeformter Verbindungsstellen und ohne den Zusatz weiterer Materialien zur Erstellung der Verbindung erzeugen lässt. Konkret weisen die dazu notwendigen Halbzeuge ein Kabel auf, in dessen Inneren sich ein oder mehrere elektrische Leiter zur Signalübertragung oder Stromleitung befinden. Zumindest an einem Ende des Kabels befindet sich die bereits erwähnte Verbindungsstelle. Das zweite Kabelende kann ebenfalls eine Verbindungsstelle aufweisen, so dass das Halbzeug ein Mittelstück bei der Fertigung einer Elektrode und eines Katheters aus mindestens drei Einzelteilen ist. Handelt es sich bei dem Halbzeug um ein Endstück, so trägt das zweite Kabelende beispielsweise Elemente, die der Wahrnehmung der späteren Funktion der Elektrode beziehungsweise des Katheters dienen.

Die am Kabelende des Halbzeugs bereitgestellte Verbindungsstelle dient der Erstellung einer dauerhaften und nicht lösbaren elektrischen Verbindung zwischen zwei Halbzeugen, die komplementäre Verbindungsstellen aufweisen. Im Bereich der Verbindungsstelle ist zu diesem Zwecke ein Teil der Oberfläche des Leiters frei zugänglich. Über diesen frei zugänglichen Bereich soll später der elektrische Kontakt zu einem zweiten Halbzeug mit komplementärer Verbindungsstelle erstellt werden. Zu beachten ist, dass der elektrische Leiter über die gesamte Länge der Verbindungsstelle nur partiell freiliegt, das heißt, zumindest eine Unterseite der dreidimensionalen Leiterstruktur wird immer durch ein Teilstück des Mantels aus dem elektrisch isolierenden thermoplastischen Material abgedeckt. Mit anderen Worten, die im Bereich der Verbindungsstelle noch vorhandenen Teile des Mantels dienen als Träger für den elektrischen Leiter. Eine Kontur und Dimensionierung der Verbindungsstellen zweier komplementärer Halbzeuge sollte daher so geschaffen sein, dass immer die mechanische Integrität der jeweiligen Verbindungsstelle gewahrt bleibt.

Wie bereits mehrfach erwähnt, ist der elektrische Leiter im Bereich der Verbindungsstelle an keiner Stelle vollständig ummantelt. Vielmehr zeigt die Verbindungsstelle eine in Längsrichtung des Kabels durchgehende Ausnehmung. Die Form dieser Ausnehmung entspricht zumindest weitgehend der Form einer Verbindungsstelle eines zweiten Halbzeugs, so dass die beiden Verbindungsstellen aufeinandergelegt (nahezu) lückenfrei die zu kontaktierenden elektrischen Leiter umschließen. Das Material im Bereich der Verbindungsstelle und in den weiteren Abschnitten des Kabels kann, muss jedoch nicht zwangsläufig dasselbe sein. Ebenso verhält es sich mit der Materialwahl des Mantels im Bereich der Verbindungsstelle und den weiteren Bereichen des Kabels. Besonders bevorzugt ist aber zumindest das Material des Mantels im Bereich der Verbindungsstelle und in den sonstigen Abschnitten des Kabels identisch.

Die einzelnen Kabel der Halbzeuge haben demnach speziell geformte Enden, die im Schlüssel-Schloss-Prinzip aufeinanderpassen und gegebenenfalls durch geeignete Umformung aufeinander laminiert werden können, so dass eine elektrisch einwandfreie und dauerhafte Verbindung entsteht. Die notwendige Verbindung von einzeln hergestellten Kabeln kann in der Folge kostengünstiger und zuverlässiger hergestellt werden.

Somit lassen sich Kabelstränge einzeln hergestellter Halbzeuge für die Fertigung von Elektroden oder Kathetern durch einen automatisierbaren thermischen Fügeprozess (gegebenenfalls unter Anwendung von Druck) permanent verbinden. Dabei können auch mehrere elektrisch voneinander isolierte Leitungen innerhalb der Kabel ohne merkliche Erhöhung des elektrischen Widerstands zuverlässig kontaktiert werden. Die entstehende Fügestelle ist hermetisch verschlossen und nicht ohne optische Hilfsmittel erkennbar.

Katheter und Elektroden für medizinische Anwendungen, die aus flexiblen Leiterplatten hergestellt sind, können somit mittels einer formschlüssigen Verbindung verlängert werden. Das erfindungsgemäße Verfahren unterscheidet sich von herkömmlichen Verbindungsverfahren auch dadurch, dass keine zusätzlichen Materialien (zum Beispiel Lötmaterial oder leitfähige Kleber) verwendet werden. Beim erfindungsgemäßen Verfahren entsteht der elektrische Kontakt durch Kontakt zwischen den Metallstrukturen im Bereich der Verbindungsstellen. Die Stabilität der elektrischen Verbindung ergibt sich durch den fest umschließenden Kunststoffkörper, der den Kontakt der Metallstrukturen dauerhaft aufrechterhält.

Vorzugsweise umfasst Schritt c) des thermischen Verschmelzens ein Aufheizen des Materials auf eine Temperatur im Bereich von 80°C bis 450°C für eine Dauer im Bereich von 1 s bis 30 min. Die Verbindungsstellen können ferner im Schritt c) oder unmittelbar nach Schritt c) mit einem Druck im Bereich von 1 bis 40 bar beaufschlagt werden. Unter den genannten Verfahrensbedingungen lässt sich die elektrische Verbindung besonders zuverlässig erstellen. Das thermische Verschmelzen des Materials kann insbesondere durch Ultraschallbehandlung erfolgen oder zumindest unterstützt werden. Nach einer weiteren bevorzugten Variante wird vor, nach oder während des Verschmelzens durch eine zusätzliche Ultraschallbehandlung die Verbindung zwischen den Kontaktflächen der beiden Leiter unterstützt.

Ferner ist bevorzugt, wenn der Leiter zumindest im Bereich der Kontaktfläche der Verbindungsstelle aus einer auf dem Mantel aufgetragenen Metallschicht besteht. Die Metallschicht kann (i) aus zumindest einem Edelmetall oder zumindest einer Edelmetalllegierung oder (ii) aus einer mit zumindest einem Edelmetall oder zumindest einer Edelmetalllegierung bedeckten Kupferleitung geformt sein. Für die genannten Zwecke eignen sich insbesondere Gold, Silber, Palladium oder deren Legierungen. Die Edelmetalle an der freien Oberfläche des Leiters verhindern die Bildung einer Oxidschicht. Insbesondere kann die Metallschicht eine Breite im Bereich von 5 bis 100 µm aufweisen. Eine Dicke der Metallschicht liegt vorzugsweise im Bereich von 0,1 bis 25 µm. Die Herstellung derartiger strukturierter Metallschichten auf beispielsweise einem Kunststoffträger stellt eine etablierte Technik dar. Insbesondere komplexe Leiterstrukturen mit einer Vielzahl von verschiedenen Kontaktflächen und Leitern können auf diese Weise in hochautomatisierten Prozessen realisiert werden.

Weiterhin ist bevorzugt, wenn die Kontaktfläche 1 µm² bis 1 mm² groß ist.

Gemäß einer weiteren Ausführungsform weist die Metallschicht vorzugsweise im Bereich der Kontaktfläche eine Erhöhung auf. Durch die Erhöhung, beispielsweise in Form eines partiell aufgetragenen Partikels (oder auch Bond Ball) aus einem hochgradig leitfähigem Metall oder einer Metalllegierung, insbesondere Kupfer, Gold oder Palladium, kann der elektrische Kontakt besonders zuverlässig und unter geringem Ausschuss erstellt werden. Dieses Partikel wird beim thermischen Verschmelzen deformiert und überbrückt so einen eventuell vorhandenen Spalt zwischen den beiden zu verbindenden Leitern. Durch den Einsatz des Partikels kann die Kontaktfläche minimiert werden.

Das thermoplastische Material des Mantels ist vorzugsweise ein Kunststoff, insbesondere ein Thermoplast mit einer Verarbeitungstemperatur von weniger als 350°C, vorzugsweise weniger als 250°C. Bevorzugt ist der Kunststoff ausgewählt aus der Gruppe umfassend Polyethylen (PE), Polypropylen (PP), Polycarbonat (PC), Polyvinylchlorid (PVC), Acrylnitril-Butadien-Styrol-Copolymer (ABS), Polyethylenterephthalat (PET), Flüssigkristallpolymere, Polyurethan, Polyetheretherketon (PEEK) und Copolymere des Polyurethans. Besonders bevorzugt ist der Kunststoff ausgewählt aus der Gruppe umfassend Flüssigkristallpolymere, Polyurethan und Copolymere des Polyurethans. Die genannten Materialien zeichnen sich durch eine besonders gute Prozessfähigkeit für das erfindungsgemäße Verbindungsverfahren aus. Ferner lassen sich auch die Verbindungsstellen der Halbzeuge besonders einfach unter Verwendung dieser Materialien herstellen. So ist beispielsweise eine Metallbeschichtung der genannten Kunststoffe zur Herstellung der elektrischen Leiter ohne weiteres möglich.

Wie bereits erwähnt, müssen die Verbindungsstellen so ausgelegt sein, dass ihre mechanische Integrität im vereinzelten Halbzeug gewahrt ist und gleichzeitig das erfindungsgemäße Verbindungsverfahren unterstützt wird. Demzufolge müssen die komplementären Verbindungsstellen zweier Halbzeuge aufeinander abgestimmt sein. Besonders zuverlässig und einfach kann dies gemäß einer ersten Ausführungsform geschehen, bei der die Konturen der Ausnehmung und des Mantels im Bereich der Verbindungstelle zusammengelegt einen Zylinder ergeben, der den Leiter umschließt, und die Ausnehmung die Form eines Kreissegments besitzt, dessen Mittelpunktswinkel α einen Wert im Bereich von 90° bis 270°, insbesondere 135° bis 225° aufweist. Bei Segmenthöhen, die kleiner als der Radius sind, kann das Volumen der Ausnehmung - mit Ausnahme des Bereichs der Kontaktfläche - auch bis zur Kreissehne begrenzt sein. Die Segmenthöhe des Kreissegments ergibt sich dann entsprechend.

In einer weiteren, besonders einfach zu realisierenden und bevorzugten Ausführungsform ist vorgesehen, dass die Konturen der Ausnehmung und des Mantels im Bereich der Verbindungstelle zusammengelegt einen Quader bilden, der den Leiter umschließt, und ein Volumen der Ausnehmung im Bereich von 75% bis 125% des Volumens des Mantels im Bereich der Verbindungstelle beträgt. Der Quader des Mantels kann insbesondere eine Dicke im Bereich von 10 bis 500 µm aufweisen.

Ferner ist bevorzugt, wenn eine Kontur der Ausnehmung nicht deckungsgleich mit einer Kontur des verbleibenden Teils des Mantels im Bereich der Verbindungsstelle ist. Gemäß dieser bevorzugten Ausführungsform ist demnach vorgesehen, dass die aufeinander passenden Verbindungsstellen zweier Halbzeuge nicht vollkommen identisch ausgelegt sind. Auf diese Weise kann erreicht werden, dass sich Halbzeuge nicht beliebig miteinander verbinden lassen, sondern nur eine in der Formgebung passende Verbindung möglich ist. Beispielsweise kann bei der obig beschriebenen zylinderförmigen Ausgestaltung von Ausnehmung und Mantel der Mittelpunktswinkel bei den beiden komplementären Halbzeugen von 180° abweichend festgelegt werden. Ebenso ist denkbar, bei der weiter oben beschriebenen quaderförmigen Verbindungsstelle das Volumen der Ausnehmung ungleich dem Volumen des Mantels im Bereich der Verbindungsstelle vorzugeben. Selbstverständlich kann die Kontur der Ausnehmung mit Hinsicht auf eine Individualisierung der Verbindungsstelle beliebig variiert werden.

Weitere bevorzugte Ausführungsformen lassen sich den Unteransprüchen und der nachfolgenden Beschreibung entnehmen.

Die Erfindung wird nachfolgend anhand von Ausführungsbeispielen und den dazugehörigen Figuren näher erläutert. Es zeigen:
- Fig. 1: eine schematische Darstellung eines Teils eines Halbzeugs im Bereich der erfindungsgemäßen Verbindungsstelle;
- Fig. 2: eine stark schematisierte Darstellung einer quaderförmigen Verbindungs-stelle gemäß einer weiteren Ausführungsform;
- Fig. 3: eine schematische Darstellung zweier komplementärer, quaderförmiger Verbindungsstellen;
- Fig. 4: eine schematische Darstellung des Verfahrens zur Herstellung der elektrischen Verbindung;
- Fig. 5: eine schematische Schnittansicht durch den Bereich der Verbindungsstelle nach Abschluss des Verbindungsverfahrens;
- Fig. 6: eine weitere alternative Ausführungsform der Verbindungsstelle; und
- Fig. 7: eine weitere schematische Darstellung einer Verbindungstelle zweier komplementärer Halbzeuge gemäß einer weiteren Ausführungsform.

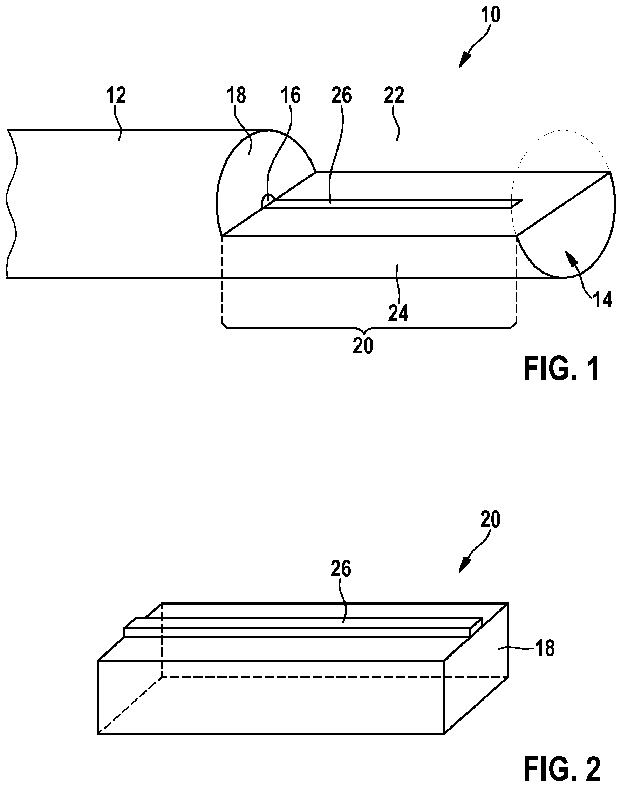

Figur 1 zeigt in stark schematisierter Weise einen Teilbereich eines Halbzeugs 10, das zur Herstellung einer flexiblen Elektrode oder eines flexiblen Katheters mit medizinischer Anwendung dient. Das Halbzeug 10 beinhaltet unter anderem ein Kabel 12 mit einem in besonderer Weise ausgestalteten Kabelende 14. Im Kabel 12 befindet sich ein elektrischer Leiter 16, der von einem Mantel 18 aus einem elektrisch isolierenden und thermoplastischen Material, wie einem Flüssigkristallpolymer oder Polyurethan, umgeben ist. Das Kabel 12 kann auch eine weitere Ummantelung besitzen, sofern dies für den jeweiligen Anwendungszweck angezeigt ist.

Wie ersichtlich, ist das Kabelende 14 Bestandteil des Kabels 12, das heißt für die weiter noch näher beschriebene Verbindungsstelle 20 wird dasselbe Mantelmaterial verwendet.

Die Verbindungsstelle 20 zeichnet sich dadurch aus, dass Teile des Mantels 18 entfernt worden sind. Demnach ergibt sich die durch die gestrichelte Linie angedeutete Ausnehmung 22, in der hier dargestellten Ausführungsform nimmt der Mantel 18 im Bereich der Verbindungsstelle 20 die Form eines Halbzylinders 24 ein. Selbstverständlich kann der Mittelpunktswinkel α auch abweichend von 180° vorgegeben werden, so dass eine Kontur der Ausnehmung 22 und des Mantels 18 im Bereich der Verbindungsstelle 20 nicht mehr identisch ist.

Auf dem Halbzylinder 24 des Mantels 18 im Bereich der Verbindungsstelle 20 ist eine Metallschicht 26 aufgetragen. Beispielsweise kann eine solche Struktur durch Metallabscheidung von Gold oder Palladium erzeugt werden. Denkbar ist auch zunächst eine Kupferschicht aufzutragen, deren Oberfläche wiederum mit Gold oder Palladium veredelt wird. Die genannten Materialien haben eine besonders hohe Leitfähigkeit.

Der Figur 2 ist stark vereinfacht lediglich eine besondere Ausführungsform der Verbindungsstelle 20 zu entnehmen, bei der der noch vorhandene Teil des Mantels 18 die Form eines Quaders einnimmt. Auf diesem Quader ist wiederum eine Metallschicht 26 aus Gold aufgetragen. Der Quader kann aus einem thermoplastischen Flüssigkristallpolymer bestehen.

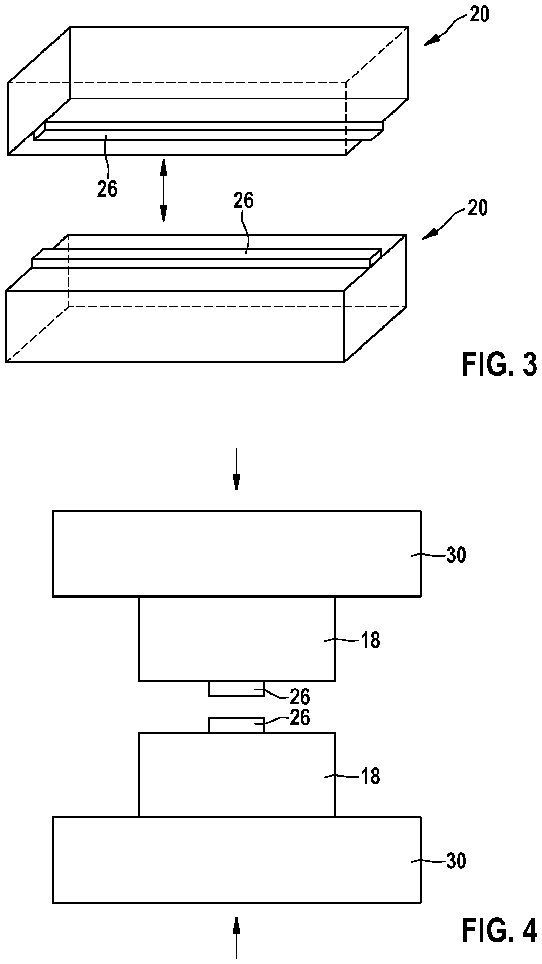

In der Figur 3 ist nun dargestellt, wie ein gleichartig ausgebautes Gegenstück zu der in Figur 2 dargestellten Verbindungsstelle 20 ausgerichtet und aufgelegt wird, so dass die Metallstrukturen an einer Stelle aufeinander zu liegen kommen und sich demnach eine elektrische Kontaktfläche zwischen den beiden Leitern ergibt. Dargestellt sind hier Verbindungsstellen 20 mit jeweils längsverlaufendem Leiter 16. Es können jedoch auch mehrere parallel verlaufende oder beliebig anders verlaufende Leiter miteinander verbunden werden. Ebenso ist denkbar, dass sich die Metallstrukturen nur an einzelnen Punkten beim Aufeinanderliegen treffen.

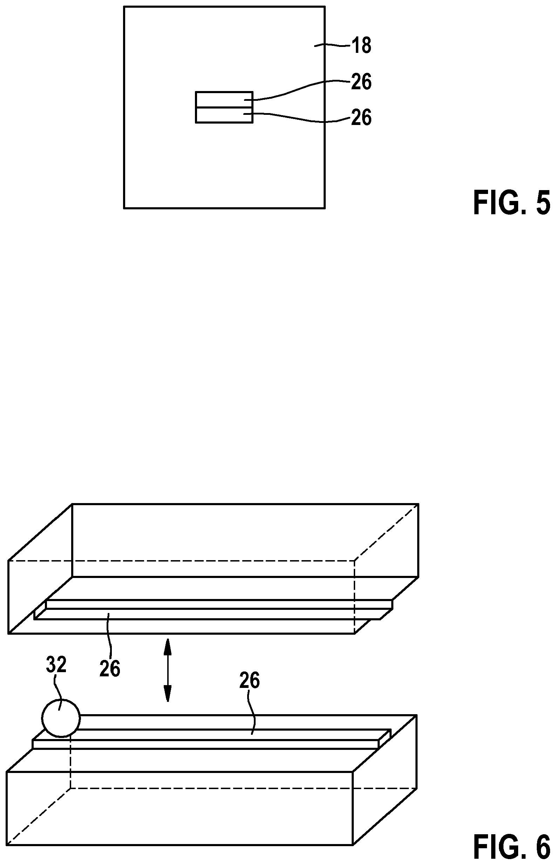

Durch ein geeignetes Werkzeug 30, beispielsweise einem Metallstempel mit integrierter Heizung, wird zur Herstellung der elektrischen Verbindung ein Druck von 1 bis 40 bar aufgebracht und das Werkzeug 30 kurzfristig von der Umgebungstemperatur auf 80°C bis 450°C erwärmt und wieder abgekühlt (siehe Figur 4). Wie ersichtlich, liegen die Kontaktflächen der beiden Leiter 16 der jeweiligen Verbindungsstellen 20 einander gegenüber. Die thermische Behandlung wird für 1 Sekunde bis 30 Minuten aufrechterhalten. Nach dem Abkühlen auf Umgebungstemperatur wird das Werkzeug 30 wieder entfernt. Das Werkzeug 30 kann eine Form aufweisen, die beim Wärmeprozess in das Kunststoffmaterial abgeformt wird. Das Werkzeug 30 kann ferner mit Teflon oder einer Keramik beschichtet sein, um eine Haftung des thermoplastischen Kunststoffs am Werkzeug 30 zu verhindern. Nach der Verpressung ergibt sich ein homogener Kunststoffkörper, in dem die Metallschichten 26 aneinander liegend eingebettet sind (siehe Figur 5).

Um die Kontaktflächen zwischen den Metallstrukturen und der Verbindungsstelle 20 zu verbessern, kann ein Goldpartikel 32 aufgesetzt werden (siehe Figur 6). Das Partikel kann dabei in einer kleinen Öffnung im Kunststoff liegen und wird beim Verpressen deformiert.

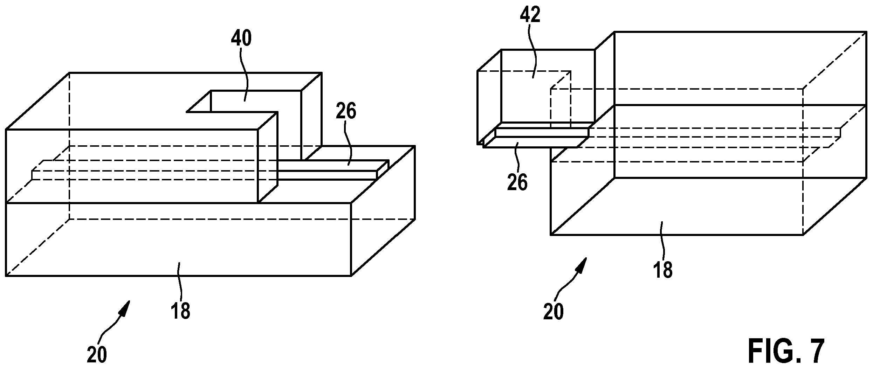

Figur 7 ist eine weitere Ausführungsform der Verbindungsstelle 20 zweier komplementärer Halbzeuge zu entnehmen. Hier weisen die beiden Verbindungsstellen 20 aufeinander passend abgestimmte Strukturen auf. So bildet die auf der linken Seite der Figur 7 dargestellte Verbindungsstelle 20 eine Nische 40 aus, zu der die auf der rechten Seite dargestellte Verbindungsstelle 20 eine entsprechende Nase 42 besitzt. Auf diese Weise kann erreicht werden, dass die Kontaktflächen der beiden Leiter 26 exakt aufeinanderliegen. Die Dimensionen tragen beispielsweise 5 bis 100 µm Breite der Metallstrukturen, 0,1 bis 25 µm Dicke der Metallstrukturen und etwa 1 µm² bis 1 mm² Größe der Kontaktfläche (Überlappung gegenüberliegender Metallschichten 26).

Die Metallschichten können chemisch, elektro-chemisch (galvanisch) oder durch Vakuumprozesse auf das Kunststoffmaterial aufgebracht werden. Weiterhin ist denkbar, Metall auf den Träger zu laminieren und mit bekannten Verfahren zu strukturieren.

### Bezugszeichenliste

- 10: Halbzeug
- 12: Kabel
- 14: Kabelende
- 16: Leiter
- 18: Mantel
- 20: Verbindungsstelle
- 22: Ausnehmung
- 24: Halbzylinder
- 26: Metallschicht
- 30: Werkzeug
- 32: Goldpartikel
- 40: Nische
- 42: Nase

## Patentansprüche

1. Verfahren zur Herstellung einer Elektrodenleitung oder eines Katheters, wobei das Verfahren die folgenden Schritte umfasst:
a) Bereitstellen eines ersten und zweiten Halbzeugs (10), wobei jedes Halbzeug (10) ein Kabel (12) mit zumindest einem elektrischen Leiter (16) umfasst, der in einem Mantel (18) aus einem elektrisch isolierenden, thermoplastischen Material eingebettet ist, wobei das Kabel (12) an zumindest einem Kabelende (14) eine Verbindungsstelle ausbildet, über die der Leiter (16) dauerhaft und unlösbar an weitere Komponenten anschließbar ist, wobei ein Teil der Oberfläche des Leiters (16) im Bereich der Verbindungsstelle (20) freiliegt und eine Kontaktfläche ausbildet;
b) Positionieren der beiden Halbzeuge (10) in einem Werkzeug (30) derart, dass die Verbindungsstellen (20) passend aufeinanderliegen, so dass sich die Kontaktflächen der beiden Leiter (16) berühren;
c) Thermisches Verschmelzen des thermoplastischen Materials im Bereich der Verbindungsstellen (20),
**dadurch gekennzeichnet, dass**
im Schritt a) der Mantel (18) im Bereich der Verbindungsstelle (20) eine in Längsrichtung des Kabels (12) durchgehende Ausnehmung (22) aufweist,
und wobei eine Kontur der Ausnehmung (22) der Verbindungsstelle (20) des ersten Halbzeugs (10) im Wesentlichen einer Kontur des Mantels (18) im Bereich der Verbindungsstelle (20) des zweiten Halbzeugs (10) entspricht und
in Schritt b) die Konturen der Verbindungsstellen (20) der beiden Halbzeuge (10) ineinandergreifen.

2. Verfahren nach Anspruch 1, bei dem Schritt c) des thermischen Verschmelzens ein Aufheizen des Materials auf eine Temperatur im Bereich von 80 °C bis 450 °C für eine Dauer im Bereich von 1 s bis 30 min umfasst.

3. Verfahren nach Anspruch 1 oder2, bei dem die Verbindungsstellen (20) im Schritt c) oder unmittelbar nach Schritt c) mit einem Druck im Bereich von 1 bis 40 bar beaufschlagt werden.

4. Verfahren nach Anspruch 1 bis 3, bei dem der Leiter (16) des Halbzeugs (10) zumindest im Bereich der Kontaktfläche der Verbindungsstelle (20) aus einer auf dem Mantel (18) aufgetragenen Metallschicht (26) besteht.

5. Verfahren nach Anspruch 4, bei dem die Metallschicht (26) des Halbzeugs (10) (i) aus zumindest einem Edelmetall oder zumindest einer Edelmetalllegierung oder (ii) aus einer mit zumindest einem Edelmetall oder zumindest einer Edelmetalllegierung bedeckten Kupferleitung geformt ist.

6. Verfahren nach Anspruch 4 oder 5, bei dem die Metallschicht (26) des Halbzeugs (10) eine Breite im Bereich von 5 bis 100 µm aufweist.

7. Verfahren nach einem der Ansprüche 4 bis 6, bei dem die Metallschicht (26) des Halbzeugs (10) eine Dicke im Bereich von 0.1 bis 25 µm aufweist.

8. Verfahren nach einem der Ansprüche 4 bis 7, bei dem die Metallschicht (26) des Halbzeugs (10) im Bereich der Kontaktfläche eine Erhöhung aufweist.

9. Verfahren nach Anspruch 1, bei dem die Kontaktfläche des Halbzeugs (10) 1 µm² bis 1 mm² groß ist.

10. Verfahren nach Anspruch 1, bei dem das thermoplastische Material des Mantels (18) des Halbzeugs (10) ausgewählt ist aus der Gruppe umfassend Polyethylen (PE), Polypropylen (PP), Polycarbonat (PC), Polyvinylchlorid (PVC), AcrylnitrilButadien-Styrol-Copolymer (ABS), Polyethylenterephthalat (PET), Polyetheretherketon (PEEK), Flüssigkristallpolymere, Polyurethan und Copolymere des Polyurethans.

11. Verfahren nach Anspruch 1, wobei bei dem Halbzeug (10) die Konturen der Ausnehmung (22) und des Mantels (18) im Bereich der Verbindungsstelle (20)zusammengelegt einen Zylinder ergeben, der den Leiter (16) umschließt, und die Ausnehmung (22) die Form eines Kreissegments besitzt, dessen Mittelpunktswinkel α einen Wert im Bereich von 90° bis 270°, insbesondere 135° bis 225° aufweist.

12. Verfahren nach Anspruch 1, wobei bei dem Halbzeug (10) die Konturen der Ausnehmung (22) und des Mantels (18) im Bereich der Verbindungsstelle (20) zusammengelegt einen Quader bilden, der den Leiter (16) umschließt, und ein Volumen der Ausnehmung (22) im Bereich von 75% bis 125% des Volumens des Mantels (18) im Bereich der Verbindungsstelle (20) beträgt.

13. Verfahren nach Anspruch 12, wobei bei dem Halbzeug (10) der Quader des Mantels (18) eine Dicke im Bereich von 10 bis 500 µm aufweist.

14. Verfahren nach Anspruch 1, wobei bei dem Halbzeug (10) eine Kontur der Ausnehmung (22) nicht deckungsgleich mit einer Kontur des verbleibenden Teils des Mantels (18) im Bereich der Verbindungsstelle (20) ist.

## Claims

1. A method for producing an electrode lead or a catheter, the method comprising the following steps:
a) providing a first and a second semi-finished product (10), each semi-finished product (10) comprising a cable (12) including at least one electrical conductor (16), which is embedded in a sheath (18) made of an electrically insulating thermoplastic material, the cable (12) on at least one cable end (14) forming a connecting point via which the conductor (16) can be permanently and non-detachably connected to further components, a portion of the surface of the conductor (16) being exposed in the region of the connecting point (20) and forming a contact surface;
b) positioning the two semi-finished products (10) in a tool (30) in such a way that the connecting points (20) are suitably disposed on top of one another so that the contact surfaces of the two conductors (16) make contact with one another;
c) thermally fusing the thermoplastic material in the region of the connecting points (20),
**characterized in that**
in step a), the jacket (18) in the region of the connecting point (20) includes a continuous recess (22) in the longitudinal direction of the cable (12),
a contour of the recess (22) of the connecting point (20) of the first semi-finished product (10) essentially corresponding to a contour of the sheath (18) in the region of the connecting point (20) of the second semi-finished product (10) and
in step b), the contours of the connecting points (20) of the two semi-finished products (10) engage one another.

2. The method according to claim 1, wherein step c) of thermal fusing includes a heating of the material to a temperature in the range of 80°C to 450°C for a duration in the range of 1 s to 30 min.

3. The method according to claim 1 or 2, wherein a pressure in the range of 1 to 40 bar is applied the connecting points (20) in step c), or immediately after step c).

4. The method according to claims 1 to 3, wherein the conductor (16) of the semi-finished product (10), at least in the region of the contact surface of the connecting point (20), is made of a metal layer (26) applied to the sheath (18).

5. The method according to claim 4, wherein the metal layer (26) of the semi-finished product (10) is formed (i) of at least one noble metal or at least one noble metal alloy, or (ii) of a copper line covered with at least one noble metal or at least one noble metal alloy.

6. The method according to claim 4 or 5, wherein the metal layer (26) of the semi-finished product (10) has a width in the range of 5 to 100 µm.

7. The method according to any one of claims 4 to 6, wherein the metal layer (26) of the semi-finished product (10) has a thickness in the range of 0.1 to 25 µm.

8. The method according to any one of claims 4 to 7, wherein the metal layer (26) of the semi-finished product (10) has an elevation in the region of the contact surface.

9. The method according to claim 1, wherein the contact surface of the semi-finished product (10) measures 1 µm² to 1 mm² in size.

10. The method according to claim 1, wherein the thermoplastic material of the sheath (18) of the semi-finished product (10) is selected from the group consisting of polyethylene (PE), polypropylene (PP), polycarbonate (PC), polyvinylchloride (PVC), acrylonitrile butadiene styrene (ABS) copolymer, polyethylene terephthalate (PET), polyether ether ketone (PEEK), liquid crystal polymers, polyurethane and copolymers of polyurethane.

11. The method according to claim 1, wherein, in the semi-finished product (10), the contours of the recess (22) and of the sheath (18), combined in the region of the connecting point (20), yield a cylinder that surrounds the conductor (16), and the recess (22) has the shape of a circular segment, having a central angle α that has a value in the range of 90° to 270°, and in particular 135° to 225°.

12. The method according to claim 1, wherein, in the semi-finished product (10), the contours of the recess (22) and of the sheath (18), combined in the region of the connecting point (20), form a cuboid that surrounds the conductor (16), and a volume of the recess (22) is in the range of 75% to 125% of the volume of the sheath (18) in the region of the connecting point (20).

13. The method according to claim 12, wherein, in the semi-finished product (10), the cuboid of the sheath (18) has a thickness in the range of 10 to 500 µm.

14. The method according to claim 1, wherein, in the semi-finished product (10), a contour of the recess (22) is not congruent with a contour of the remaining portion of the sheath (18) in the region of the connecting point (20).

## Revendications

1. Procédé de fabrication d'une ligne d'électrode ou d'un cathéter, le procédé comprenant les étapes suivantes :
a) de mise au point d'un premier et d'un deuxième produit semi-fini (10), où chaque produit semi-fini (10) comprend un câble (12) avec au moins un conducteur (16) électrique qui est incorporé dans une gaine (18) à base d'un matériau thermoplastique isolant électriquement, où le câble (12) forme au niveau d'au moins une extrémité de câble (14) une jonction, au moyen de laquelle le conducteur (16) peut être raccordé de manière durable et inamovible à d'autres composants, où
une partie de la surface du conducteur (16) est exposée dans la zone de la jonction (20) et forme une surface de contact ;
b) de positionnement des deux produits semi-finis (10) dans un outil (30) de telle manière que les jonctions (20) se superposent exactement de sorte que les surfaces de contact des deux conducteurs (12) se touchent ;
c) de fusion thermique du matériau thermoplastique dans la zone des jonctions (20),
**caractérisé en ce que**
dans l'étape a), la gaine (18) dans la zone de la jonction (20) présente un évidement (22) allant de part en part dans la direction longitudinale du câble (12),
et où un contour de l'évidement (22) de la jonction (20) du premier produit semi-fini (10) correspond essentiellement à un contour de la gaine (18) dans la zone de la jonction (20) du deuxième produit semi-fini (10), et
dans l'étape b), les contours des jonctions (20) des deux produits semi-finis s'interpénètrent.

2. Procédé selon la revendication 1, chez lequel l'étape c) de fusion thermique comprend un réchauffement du matériau à une température dans la plage de 80 °C à 450 °C pour une durée dans la plage de 1 s à 30 min.

3. Procédé selon la revendication 1 ou 2, chez lequel les jonctions (20) sont soumises à une pression dans la plage de 1 à 40 bar dans l'étape c) ou immédiatement après l'étape c).

4. Procédé selon la revendication 1 à la revendication 3, chez lequel le conducteur (16) du produit semi-fini (10) est constitué au moins dans la zone de la surface de contact de la jonction (20) d'une couche métallique (26) rapportée sur la gaine (18).

5. Procédé selon la revendication 4, chez lequel la couche métallique (26) du produit semi-fini (10) est formée (i) à base d'au moins un métal noble ou d'au moins un alliage de métal noble ou (ii) à base d'une conduite en cuivre revêtue avec au moins un métal noble ou avec au moins un alliage de métal noble.

6. Procédé selon la revendication 4 ou 5, chez lequel la couche métallique (26) du produit semi-fini (10) présente une largeur dans la plage de 5 à 100 µm.

7. Procédé selon l'une des revendications 4 à 6, chez lequel la couche métallique (26) du produit semi-fini (10) présente une épaisseur dans la plage de 0,1 à 25 µm.

8. Procédé selon l'une des revendications 4 à 7, chez lequel la couche métallique (26) du produit semi-fini (10) présente une élévation dans la zone de la surface de contact.

9. Procédé selon la revendication 1, chez lequel la surface de contact du produit semi-fini (10) a une taille de 1 µm² à 1 mm².

10. Procédé selon la revendication 1, chez lequel le matériau thermoplastique de la gaine (18) du produit semi-fini (10) est choisi dans le groupe comprenant le polyéthylène (PE), le polypropylène (PP), le polycarbonate (PC) le chlorure de polyvinyle (PVC), le copolymère acrylonitrile-butadiène-styrène (ABS), le polyéthylène téréphtalate (PET), la polyétheréthercétone (PEEK), des polymères de cristaux liquides, le polyuréthane et des copolymères du polyuréthane.

11. Procédé selon la revendication 1, dans lequel, chez le produit semi-fini (10), les contours de l'évidement (22) et de la gaine (18) assemblés forment, dans la zone de la jonction (10), un cylindre qui entoure le conducteur (16), et l'évidement (22) possède la forme d'un segment circulaire dont l'angle au centre α présente une valeur dans la plage de 90 ° à 270 °, notamment de 135 ° à 225 °.

12. Procédé selon la revendication 1, dans lequel, chez le produit semi-fini (10), les contours de l'évidement (22) et de la gaine (18) assemblés forment, dans la zone de la jonction (10), un cuboïde qui entoure le conducteur (16), et un volume de l'évidement (22) s'élève dans la plage de 75 % à 125 % du volume de la gaine (18) dans la zone de la jonction (20).

13. Procédé selon la revendication 12, dans lequel, chez le produit semi-fini (10), le cuboïde de la gaine (18) présente une épaisseur dans la plage de 10 à 500 µm.

14. Procédé selon la revendication 1, dans lequel, chez le produit semi-fini (10), un contour de l'évidement (22) ne se superpose pas exactement avec un contour de la partie restante de la gaine (18) dans la zone de la jonction (20).
